# EUROPEAN PATENT SPECIFICATION

(11) **EP 4 280 937 B1**
(45) Date of publication and mention of the grant of the patent: **25.11.2026**
(21) Application number: 22701577.3
(22) Date of filing: 19.01.2022
(51) Int. Cl.: A61B 5/00, A61B 5/369

(54) **A SLEEP ANALYZER AND ASSOCIATED SYSTEMS AND METHODS**
SCHLAFANALYSATOR UND ZUGEHÖRIGE SYSTEME UND VERFAHREN
ANALYSEUR DE SOMMEIL ET SYSTÈMES ET PROCÉDÉS ASSOCIÉS

(30) Priority: 19.01.2021 EP 21305058
(43) Date of publication of application: 29.11.2023
(73) Proprietor: Institut national de la santé et de la recherche médicale (INSERM), 75013 Paris (FR); Centre Hospitalier Universitaire de Poitiers, 86000 Poitiers (FR); Université de Poitiers, 86034 Poitiers Cedex (FR); Somno Engineering SAS, 86961 Chasseneuil-du-Poitou (FR)
(72) Inventor: DROUOT, Xavier, 86073 Poitiers Cedex 9 (FR); DE LA BELLEISSUE, Maud Nicol, 37300 Joué-lès-Tours (FR)
(74) Representative: Icosa
(86) International application number: PCT/EP2022/051140
(87) International publication number: WO 2022/157199

(56) References cited:
- WO-A1-2015/111012
- WO-A1-2020/140122
- JP-A- H0 453 527
- US-A1- 2011 275 960
- US-A1- 2018 333 558

## Description

### TECHNICAL FIELD OF THE INVENTION

The present invention concerns a sleep analyzer. The present invention also deals with a system of monitoring, an healthcare room and an healthcare installation equipped with said sleep analyzer, notably intensive room and installation. The present invention also concerns corresponding method for analyzing the sleep of a subject and method for monitoring the sleep of a subject.

### BACKGROUND OF THE INVENTION

Poor sleep is a significant issue globally and impacts up to 60% of the adult population.

A large number of publications have thus been dedicated to issues associated with sleep. The research work carried out in such publications has led to show a correlation between an insufficient rest and overall mood, underperformance at the work place, causing an accident, obesity or diabetes.

In the sleep publication, a new field of research is emerging and concerns the impact of poor sleep on people fallen in coma.

In particular, such impact is analysed in an article by Martin Dres et al. whose title is "Sleep and Pathological Wakefulness at the Time of Liberation from Mechanical Ventilation (SLEEWE)" and was published in the review American Journal of Respiratory and Critical Care Medicine (volume 199, number 9, May 1st, 2019).

More specifically, in this article, the existence of abnormal patterns of sleep and wakefulness in mechanically ventilated patients is studied. It is shown that patients who pass spontaneous breathing trials (SBT) and are extubated reach higher levels of wakefulness as indicated by the odds ratio products (ORP), suggesting abnormal wakefulness in others. The hemispheric ORP correlation is much poorer in patients who fail an SBT.

This article is thus a proof that it is desirable to be able to monitor sleep in intensive healthcare unit.

However, the method and system used in the article require very specific material and the presence of sleep specialists, which renders the use of such monitoring method and system very difficult to implement.

Document US2011275960 describes other sleep monitoring devices and methods: This document specifically discloses an entrance information system comprises a sleep detector for detecting the sleep stage of a first person in a sleeping room. This entrance instruction system for a sleeping room may be used in a hospital. An entrance instruction for the sleeping room is allocated in dependence of the sleeping signal of the sleeping detector and issued by a output unit.

### SUMMARY OF THE INVENTION

The invention is defined by the appended claims.

There is a need for methods and/or systems involved in the monitoring of the sleep of a subject in an healthcare room, notably an intensive one, which are easier to implement. To this end, the specification describes a sleep analyzer, the sleep analyzer comprising a housing, a sleep recorder adapted to sense physical values relative to the sleep of a subject, the sleep recorder including an electrode adapted to record the electrical activity of the subject. The sleep analyzer further comprises a calculator adapted to deduce the sleep state of the subject from the sensed physical values, and a transceiver adapted to transmit a data obtained from the deduced state of the subject to an external receiver. The sleep recorder, the calculator and the transceiver being in the housing.

According to further aspects of this sleep analyzer, which are advantageous but not compulsory, the sleep analyzer might incorporate one or several of the following features, taken in any technically admissible combination:
- the sleep analyzer comprises a sensor for sensing a parameter relative to the environment of the sleep analyzer.
- the sleep analyzer comprises a sensor for sensing a parameter relative to the subject, notably to the breath of the subject, the movement of the subject or the brain activity of the subject.
- the sleep recorder comprises an accelerometer.
- the housing comprises walls defining an inner volume inferior to 50 square centimeters.
- the sleep analyzer is provided with a power supply, the power supply being in the housing.

The specification also describes a system for monitoring the sleep of a subject comprising a sleep analyzer as previously described, a receiver adapted to receive the data obtained from the deduced state of the subject, and an interface unit adapted to indicate at least one of the received data.

According to further aspects of this system for monitoring, which are advantageous but not compulsory, the system for monitoring might incorporate one or several of the following features, taken in any technically admissible combination: the interface unit is a human machine interface.
- the interface unit comprises at least one of a tablet and a light indicator.
- the system further comprises an environment controller, the environment controller being adapted to control at least one parameter of the environment.
- the environment controller is adapted to control at least one parameter of the environment based on the received data.
- the environment controller comprises at least one of element, the element being chosen among a light source, a loudspeaker, an air controller and an air-conditioning. The specification also relates to a healthcare room, notably an intensive one, equipped with a system for monitoring the sleep of a subject as previously described.

The specification further describes a healthcare installation, notably an intensive one, comprising several healthcare rooms, at least one of the healthcare room being as previously described.

The specification also relates to a method for analyzing the sleep of a subject with a sleep analyzer, the sleep analyzer comprising a housing, a sleep recorder, a calculator and a transceiver, the sleep recorder comprising an electrode, the sleep recorder, the calculator and the transceiver being in the housing, the method for analyzing comprising sensing physical values relative to the sleep of the subject by using the sleep recorder, the sensing comprising recording the electrical activity of the subject with the electrode, deducing the sleep state of the subject from the sensed physical values by the calculator, and transmitting with the transceiver a data obtained from the deduced state of the subject to an external receiver.

The specification further describes a method for monitoring the sleep of a subject with a system for monitoring the sleep of the subject, the system of monitoring comprising a sleep analyzer, a receiver and an interface unit, the sleep analyzer comprising a housing, a sleep recorder, a calculator and a transceiver, the sleep recorder comprising an electrode, the sleep recorder, the calculator and the transceiver being in the housing, the method for monitoring comprising carrying out the steps of a method for analyzing as previously described, receiving the data obtained from the deduced state of the subject by the receiver, and indicating at least one of the received data with the interface unit

### BRIEF DESCRIPTION OF THE DRAWINGS

The invention will be better understood on the basis of the following description, which is given in correspondence with the annexed figures and as an illustrative example, without restricting the object of the invention which is defined by the appended claims. In the annexed figures:
- figure 1 is a schematic view of an healthcare intensive installation comprising a room equipped with a system for monitoring the sleep of a patient which is in the room;
- figure 2 is a schematic representation of the system for monitoring of figure 1 which notably comprises a sleep analyzer;
- figure 3 is a schematic representation of the components of a sleep analyzer.

### DETAILED DESCRIPTION OF SOME EMBODIMENTS

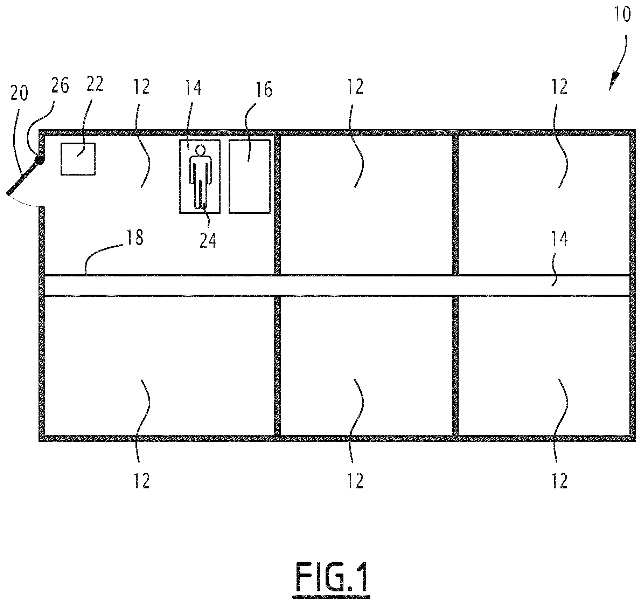

An intensive healthcare installation 10 is shown on figure 1. The intensive healthcare installation 10 comprises six intensive healthcare rooms 12, which are linked by a corridor 14.

It should be understood that what follows can also be applied to any kind of healthcare installation or healthcare room.

By clarity, only the elements of one room 12 are visible on figure 1.

The room 12 comprises an hospital bed 14, medical apparatuses 16, a door 18, a window 20 and a system for monitoring 22 the sleep of a subject.

In what follows, the hospital bed 14 will be simply referred to as bed 14 and the system for monitoring 22 the sleep of a subject will be simply referred to as the system for monitoring 22.

In figure 1, a subject 24 is lying on the bed 14.

The subject 24, which is represented in figure 1 , is a human.

More generally, the subject 24 is a living subject and notably an animal.

As a specific example, the subject 24 is a mammal.

In what follows, the subject 24 is simply named "patient 24".

The medical apparatuses 16 are apparatuses used for the care of the patient 24.

The medical apparatuses are, for instance, a perfusion and an apparatus for recording physical values of the patient 24 (cardiac pace) and an artificial respirator.

The door 18 is the entrance of the room 12.

The window 20 is an aperture towards the exterior, which provides the patient 24 with natural light.

The window 20 is provided with a window shutter 26, which is adapted to block the propagation of natural light.

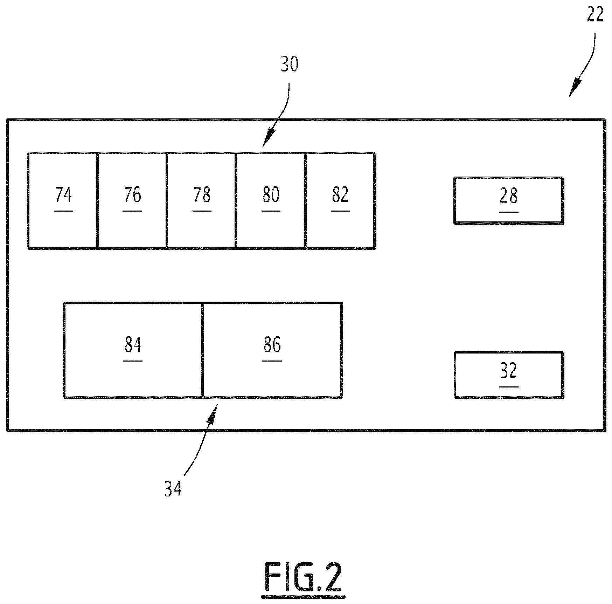

The system for monitoring 22 is schematically represented on figure 2.

The system for monitoring 22 comprises a sleep analyzer 28, an environment controller 30, a receiver 32 and an interface unit 34.

The system for monitoring 22 is a system adapted to control the sleep of the patient 24.

According to the embodiments, the system for monitoring 22 is a system for providing information relative to the sleep of the patient 24 or the system for monitoring 22 is a system for sleep management or promotion.

Notably, in another context, the system for monitoring 22 is a system for assisting a user to fall asleep.

The sleep analyzer 28 is a device adapted to analyze the sleep of the patient 24.

By analyzing the sleep of the patient 24, it is meant that data relative to the sleep of the patient 24 is determined. For instance, a data relative to the sleep is to obtain the different stages of a sleep of the patient 24.

Sleep can be characterized by three distinct stages (phases) which change throughout the night. These stages may be understood as follows:
- Stage 1 ("N1"): o Transition between being awake and being asleep. o You lose awareness of your surroundings (feels like drowsiness when you are not completely awake), and can be easily woken from this state. o May experience generalized or localized muscle contraction associated with vivid visual imagery. o Sleep onset usually lasts 5-10 minutes.
- Stage 2 ("N2"): o Sleeping, but not particularly deeply (easy to wake from this stage). o Usually lasts 10-25 minutes at a time. o Typically, you spend about half the night sleeping in this state. o Your heart rate, breathing, and brain activity slows down in this sleep stage and your body completely relaxes
- Stages 3 ("N3") : o Deep, slow wave sleep (SWS). This is believed to be the time where your body renews and repairs itself. o After falling asleep it might take up to half an hour to reach this deepest part of your sleep. It takes far more effort to wake you up. o Your breathing becomes more regular, blood pressure falls, and pulse rate slows. o The amount of deep sleep varies with age. As you get older, there is a decrease in deep sleep (and increase in lighter sleep). You tend to sleep for a shorter length of time as you age. Therefore, you are more likely to wake up during the night as you get older (i.e., you are in light sleep for longer, from which you can more easily be disturbed by noise, movement of a bed partner, discomfort etc.). This is normal, and most older adults continue to enjoy their sleep.
- Stage R ("Rapid eye movement Sleep", REM Sleep) o REM sleep is a particular sleep in which brain activity is high, approaching wake levels. Dreams and oneiric activities occur during REM sleep. Loss of REM sleep is also associated with fatigue and somnolence. REM sleep occur at the end of sleep cycle and is predominant in the second part of the night.

Other stages are defined for patient 24 fallen in the coma. These stages are often named atypical sleep.

In intensive care unit (ICU) patients, normal feature of wake electroencephalogram (EEG) and sleep EEG are replaced by a different sleep, called "pathological wakefulness" and "atypical sleep". These are wake and sleep states but features of EEG are quite different from normal. In particular, K complex and sleep spindles are rare or absent in atypical sleep. In addition, in pathological wake, background EEG frequency is slowed and less reactive to external stimuli. These both states are of major importance in ICU patients because they are both associated with a worst outcome in ICU. Then, the diagnosis of these atypical wake and sleep EEG features and the quantification of these states are crucial for intensivist.

Such data relative to the stage enables to obtain a hypnogram which is the graph presenting the evolution of the stage with time.

Another example of data is a data that can be extracted from such hypnogram is the presence of atypical sleep, the number of arousals from sleep, the duration of sleep and the duration of deep sleep.

According to another example, the data is a sleep score.

A sleep score is an index value representative of the quality of the sleep.

As an example, a sleep score may be the ratio of the time spent in stage N3 over the time spent in stages N1 and N2.

Generally, an analysis of the sleep of the patient 24 may lead to obtain one of the previous data or several of the previous data.

In this example, the sleep analyzer 28 provides with the different stages of the sleep of the patient 24.

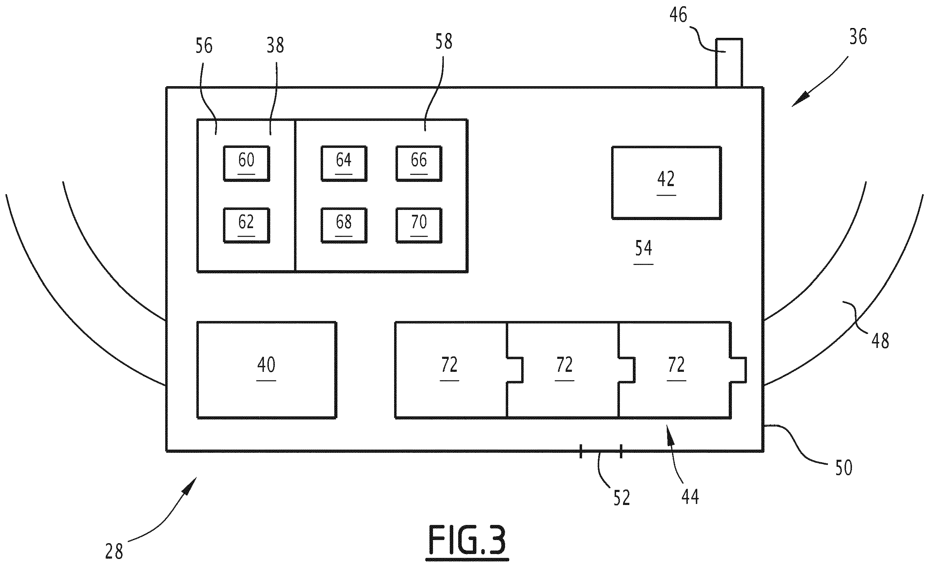

Figure 3 is a schematic view of such sleep analyzer 28, which comprises a housing 36, a sleep recorder 38, a calculator 40, a transceiver 42, a power supply 44, a light element 46 and fasteners 48.

The housing 36 comprises walls 50 and a refilling port 52.The housing 36 is quite small since it has an external volume inferior to 65 cm³.

More generally, an external volume for the housing 36 comprised between 50 cm³ and 100 cm³ is desirable.

The walls 50 are made in a material, which is resistant to bumps, water projections and to disinfection agent. More specifically, the material enables that the housing 36 resists to a fall from height of the bed 14 or from an adult hand (bump resistance).

The material also enables that the housing 36 resists to water splashing and manual friction with disinfecting agent, notably applied by a wet fabric.

The material does not necessarily resist to being immerged or to magnetic field, as can be the case when carrying out a magnetic resonance imaging (MRI).

On the walls 50, data, notably traceability data, are provided.

For instance, a label with the data written on it are glued on the walls 50.

Inventory number, product name, product version or certification reference are examples of such traceability data.

In addition, each wall 50 has an external surface, which is smooth.

This mean that the surface is deprived of whole or groove so as to be easily cleaned.

The walls 50 delimit an inner volume 54 of the housing 36.

The sleep recorder 38, the calculator 40, the power supply 44 and the transceiver 42 are in the housing 36.

More precisely, the sleep recorder 38, the calculator 40 and the transceiver 42 are in the inner volume 54.

The inner volume 54 is inferior or equal to 50 square centimeters (cm2 ), preferably inferior or equal to 40 cm2 .

In the specific example, the housing 36 has the form of a parallelepiped box with a length of 7 centimeters (cm), a thickness of 2 cm and a width of 4.5 cm.

The material and the shape of the housing 36 are chosen so that the weight of the housing 36 be inferior or equal to 100 grams, preferably inferior or equal to 80 grams.

The material and the shape of the housing 36 are chosen so that the weight of the housing 36 be superior or equal to 50 grams in order to ensure robustness of the sleep analyzer 28.

The refilling port 52 is a USB port 52 linked to the power supply 44.

The USB port 52 enables to refill the power supply 44.

The sleep recorder 38 is adapted to sense physical values relative to the sleep of the patient 24.

In other words, the sleep recorder 38 is adapted to record parameters relative to the sleep of the patient 24.

The physical values are either values relative to the patient 24 or values relative to the environment of the patient 24.

The breath of the patient 24, the movement of the patient 24, the heart rate patterns of the patient 24, the wish to sleep of the patient 24, the electromyogram activity of the patient 24 or the cerebral activity of the patient 24 are examples of values relative to the patient 24.

The intensity of light or the intensity of sound in the environment of the patient 24 are examples of values relative to the environment of the patient 24.

In the present case, the sleep analyzer 28 comprises two first sensors 56 sensing a parameter relative to the patient 24 and four second sensors 58 sensing a parameter relative to the environment of the sleep analyzer 28 (which is the environment of the patient 24).

The sleep recorder 38 is thus a set of sensors 56 and 58.

In the present case, each sensor 56, 58 is positioned on the same support for instance a printed circuit board (often named PCB).

The first sensors 56 are the accelerometer 60 and the electrode 62.

The accelerometer 60 enables to access data relative to the movement of the patient 24.

The sleep recorder 38 includes an electrode 62 adapted to record the electrical activity of the patient 24 and more precisely the cerebral activity.

In the example of figure 3, the second sensors 58 are a light sensor 64, a microphone 66, a temperature sensor 68 and an air sensor 70.

For such environment sensors 58, each sensor 58 is calibrated so as to deduce from the value measured inside the inner volume 54 the real value.

The light sensor 64 is adapted to measure the light intensity.

The microphone 66 is a sound level sensor adapted to measure the sound intensities in the environment.

The temperature sensor 68 is adapted to measure a mean temperature of the room 12.

The air sensor 70 is adapted to measure a parameter relative to the air in the room 12, such as a parameter representative of the pollution of the air and the humidity rate.

The calculator 40 is electronic circuitry adapted to manipulate and/or transform data represented by electronic or physical quantities in registers of the calculator 40 and/or memories in other similar data corresponding to physical data in the memories of the registers or other kinds of displaying devices, transmitting devices or memoring devices.

The calculator 40 is adapted to deduce data relative to the sleep of the patient 24 from one or several sensed physical values.

The calculator 40 is adapted to determine the sleep stages of a patient 24. The transceiver 42 is adapted to transmit a data deduced by the calculator 40 to an external receiver.

The transceiver 42 is adapted to operate according to a wireless connection with a range of at least 5 meters.

For instance, the transceiver 42 uses a Bluetooth protocol, notably a Bluetooth low energy protocol.

The power supply 44 is a rechargeable battery.

Such power supply 44 ensures autonomy since no direct power supply is required.

In operating, the power supply 44 provides at least one day autonomy to the sleeping analyzer.

The power supply 44 is adapted to provide with a power inferior to 5V in Direct Current (DC).

For instance, in the current example, the power supply 44 comprises several rechargeable batteries 72.

The battery are, for instance, lithium-ion battery.

The light element 46 is an indicator of the functioning of the sleep analyzer 28.

For instance, the light element 46 is an indication of the state of charge of the power supply 44.

In the example of figure 1 , the light element 46 is green when the state of charge is sufficient for the sleep analyzer 28 to operate and is red when the state of charge is insufficient for the sleep analyzer 28 to operate.

In variant, the light element 46 is orange when the state of charge is sufficient but close to the insufficient threshold. This indicates that a refilling of the power supply 44 is needed.

The fasteners 48 are adapted to fasten the housing 36 on the patient 24.

More precisely, the fasteners 48 are adapted to fasten the housing 36 on the head of the patient 24.

In position, the housing 36 is in contact with the patient 24's scalp and the fasteners 48 is surrounding the patient 24's head.

For this, the fasteners 48 are mechanical fasteners.

In the present example, the fasteners 48 are bands.

The fasteners 48 are made in extensible material.

The environment controller 30 is adapted to control at least one parameter of the environment.

In this specific example, the environment controller 30 is adapted to control at least one parameter of the environment based on the received data. The environment controller 30 comprises a shutter control 74, a light source 76, a loudspeaker 78, an air controller 80 and an air-conditioning 82.

In variant, the environment controller 30 comprises only one, two or three of the previous elements.

The receiver 32 is adapted to receive the data obtained from the deduced state of the patient 24.

The receiver 32 is not situated in the housing 36 of the sleep analyzer 28 and is thus an external receiver 32.

The interface unit 34 adapted to indicate at least one of the received data.

According to the example, the interface unit 34 comprises a tablet 84 and a light indicator 86.

Any interface human machine may be consider to constitute the interface unit 34.

The operating of the sleep analyzer 28 is now described in reference to an example of carrying out of a method for monitoring the sleep of the patient 24.

Such method for monitoring the sleep of the patient 24 is named monitoring method in the remainder of the specification.

In this example, the monitoring method comprises a phase of analyzing, a phase of indicating and a phase of improving.

The phase of analyzing corresponds to a method for analyzing the sleep of the patient 24 with the sleep analyzer 28.

Such method for analyzing is named analyzing method in what follows.

The phase of analyzing comprises a step for sensing and a step for deducing.

During the step of sensing, the sleep recorder 38 senses physical values relative to the sleep of the patient 24.

In the current case, this means that all the values sensed by the sleep recorder 38 are acquired.

In particular, the electroencephalography signal recorded during the sleep of the patient 24 is acquired.

In such context, the sleep of the patient 24 is the time wherein the patient 24 is trying to sleep, for instance by switching the light off.

In the present example, the sleep recorder 38 is positioned on the patient 24 to acquire the signal from the forehead of the patient 24.

The sleep recorder 38 acquires the electroencephalography signal at a pace superior to 200 Hz.

In addition, only one a single EEG channel is sufficient to obtain the desired data. During the step for deducing, the sleep state of the subject is deduced from the sensed physical values by the calculator 40.

The calculator 40 provides with such analysis in real time.

Any technique adapted to obtain the sleep state from physical values can be considered at this stage. The calculator 40 is also adapted to determine the states of "pathological wakefulness" and "atypical sleep", which were previously defined.

In addition, the calculator 40 may determine a sleep score.

Some techniques are notably known from an article by L. Florillo et al. entitled « Automated sleep scoring: A review of the latest approaches », which was published in 2019 in Sleep Medecine Review.

During the phase of indicating, the transceiver 42 transmits the data obtained from the deduced state of the patient 24 to the receiver 32.

The receiver 32 then receives the data obtained from the deduced state of the patient 24 sent by the transceiver 42.

The interface unit 34 then indicates at least one of the received data.

The indication by the interface unit 34 is understandable by each person without any specific training. Notably, a family member coming to visit the patient 24 will understand the indication provided by the interface unit 34.

In the present case, the light indicator 86 is adapted to take three colors: a green one, a red one and an orange one.

The light indicator 86 takes the green color when one of the following conditions is fulfilled:
- the determined stage is the phase N1 for more than five minutes,
- the determined stage is a mix of the phase N1 and of phase N2 over the previous five minutes, or
- the determined stage is the phase N2 over the previous five minutes.

The light indicator 86 takes the red color when the determined stage is the phase N3 or when the determined stage is the phase N2 over more than the previous ten minutes.

In each of the other cases, the light indicator 86 takes the orange color.

Preferably, the light indicator 86 is adapted to take only two colors: one corresponding to a "do not wake up" signal and one corresponding to an "awake signal".

In addition, the light indicator 86 and/or the calculator 40 according to the considered embodiments may be adapted according to the following elements. For this, the light indicator 86 may be provided with computer means. As a first element, no data is indicated when detecting the onset of a sleep episode, the detection of intra-sleep arousal episodes, or the detection of sleep coming out. The light indicator 86 displays the "do not wake up" signal once the current episode includes 60% of episodes of sleep among 6 consecutive epochs (here an epoch correspond to a 30s time period).

As a second element, certain intra-sleep arousal episodes are detected but the light indicator 86 do not indicate them because they are likely not to be prolonged and not to compromise the total length of the sleep episode.

More generally, the indication the "do not wake up" signal by the light indicator 86 takes into account the probability of returning to sleep (probability that the patient has to go back to sleep after waking up.

Intra-sleep arousal episodes of less than 5 minutes (continuous) are not signaled considered as triggering the indication the "do not wake up" signal because the probability of the patient 24 going back to sleep is high (and the probability of waking up permanently is low) if the patient 24 has been sleeping for a defined period of time.

Likewise, the longer a sleep episode, the more gradual the exit from this episode (waking up) is. Therefore, after a long sleep episode (for instance more than 20 minutes) an "awake" signal is only displayed by the light indicator 86 if the patient is awake for at least x consecutive minutes (x depends on the duration of the sleep episode).

These data are of major importance for the sleep of patients in emergency unit (and are specific to these patients 24): in fact, their sleep is very discontinuous and is a sum of very short episodes (less than a few minutes, notably less than 5 minutes), of episodes of medium duration (for example, between 5 minutes and 10 minutes) and longer episodes (for example, superior to 15 minutes), the latter being the only restorative episodes.

As a summary, the light indicator 86 and/or the calculator 40 are adapted to emit a signal depending on the length of the previous episodes, this length being differentiated in three categories: very short, medium and long.

In addition, the light indicator 86 and/or the calculator 40 are adapted to emit a signal depending on the ratio between long episodes and very short episodes.

It will appears for the person skilled in the art that what has just been described can be generalized for any kind of symbol. For instance, instead of colors, the indicator 86 may use a cross for a "do not wake up" signal and a circle for an "awake signal". Alternatively, the symbol may be words, emoji or closed/open eyes.

The tablet 84 displays several boxes of information. As an example, the tablet 84 has an artefact box in which it is indicated whether artefacts are presents for more than an interval of time preferably comprised between 3 and 15 minutes.

The tablet 84 also has a sleep parameter box in which, for instance, the hypnogram is displayed and the index score is also displayed.

Any combination of the previously mentioned sleep data can be displayed in the sleep parameter box.

The tablet 84 also has an environmental box in which, for instance, the values sensed by the environmental sensors 58 of the sleep analyzer 28 are displayed.

During the phase of improving, the different parameters are taken into account by the medical personal for improving the sleep quality of the patient 24.

For instance, the medical personal refrains to enter in the room 12 when the light indicator 86 indicates the red color and goes to another room 12.

The medical personal can also vary the environmental parameter to promote the sleep of the patient 24 by using any one of the shutter control 74 (for reducing the light intensity), a light source 76 (for the light intensity), a loudspeaker 78 (for sound controlling or emitting relaxing sounds), an air controller 80 (for controlling the level of pollution) and an air- conditioning 82 (for controlling the temperature of the air).

For instance, the temperature of the room 12 can be reduced.

In the present case, the different environment parameters are controlled by the environment controller 30 but manually a medical personal can adjust the environment parameters if necessary.

In addition, the sleep parameter box may be used by the doctor for diagnosis purpose.

Such method therefore enables to control in an efficient way the sleep of the patient 24.

This control is achieved in a very simple way since no trained personal is needed and since the additional material needed is very simple.

Only the system for control is required.

In other word, it has been described methods and/or systems involved in the monitoring of the sleep of a subject in an intensive healthcare room 12 which are easier to implement.

In addition, such element can also be used for other contexts.

As an example of other context, the apparatus used for implementing the determined method may be very different.

For instance, one may consider that the apparatus is computer interacting with a computer program product. The interaction between the computer program product and the computer enables to carry out the determined method, which is thus a computer- implemented method.

The computer is a desktop computer. In variant, the computer is a rack-mounted computer, a laptop computer, a tablet computer, a PDA or a smartphone.

In the case of figure 1 , the computer comprises a calculator, a user interface and a communication device.

As specific examples, the calculator comprises a monocore or multicore processor (such as a CPU, a GPU, a microcontroller and a DSP), a programmable logic circuitry (such as an ASIC, a FPGA, a PLD and PLA), a state machine, gated logic and discrete hardware components.

The calculator comprises a data-processing unit which is adapted to process data, notably by carrying out calculations, memories adapted to store data and a reader adapted to read a computer readable medium.

The user interface comprises an input device and an output device.

The input device is a device enabling the user of the computer to input information or command to the computer.

The input device is a keyboard. Alternatively, the input device is a pointing device (such as a mouse, a touch pad and a digitizing tablet), a voice-recognition device, an eye tracker or a haptic device (motion gestures analysis).

The output device is a graphical user interface, which is a display unit adapted to provide information to the user of the computer.

The output device is a display screen for visual presentation of output. In other embodiments, the output device is a printer, an augmented and/or virtual display unit, a speaker or another sound generating device for audible presentation of output, a unit producing vibrations and/or odors or a unit adapted to produce electrical signal.

In a specific embodiment, the input device and the output device are the same component forming man-machine interfaces, such as an interactive screen.

The communication device enables unidirectional or bidirectional communication between the components of the computer. For instance, the communication device is a bus communication system or an input/output interface.

The presence of the communication device enables that, in some embodiments, the components of the computer be remote one from another.

The computer program product comprises a computer readable medium.

The computer readable medium is a tangible device that can be read by the reader of the computer. Notably, the computer readable medium is not transitory signals per se, such as radio waves or other freely propagating electromagnetic waves, such as light pulses or electronic signals.

Such computer readable storage medium is, for instance, an electronic storage device, a magnetic storage device. an ontical storage device, an electromagnetic storage device, a semiconductor storage device or any combination thereof.

As a non-exhaustive list of more specific examples, the computer readable storage medium 48 is a mechanically encoded device such a punchcards or raised structures in a groove, a diskette, a hard disk, a ROM, a RAM, an EROM, an EEPROM, a magnetic-optical disk, a SRAM, a CD-ROM, a DVD, a memory stick, a floppy disk, a flash memory, a SSD or a PC card such as a PCMCIA.

A computer program is stored in the computer readable storage medium 48. The computer program comprises one or more stored sequence of program instructions.

Such program instructions when run by the data-processing unit, cause the execution of steps of any method that has been described or will be described below.

For instance, the form of the program instructions is a source code form, a computer executable form or any intermediate forms between a source code and a computer executable form, such as the form resulting from the conversion of the source code via an interpreter, an assembler, a compiler, a linker or a locator. In variant, program instructions are a microcode, firmware instructions, state-setting data, configuration data for integrated circuitry (for instance VHDL) or an object code.

Program instructions are written in any combination of one or more languages, such as an object oriented programming language (FORTRAN, C++, JAVA, HTML), procedural programming language (language C for instance).

Alternatively, the program instructions is downloaded from an external source through a network, as it is notably the case for applications. In such case, the computer program product comprises a computer-readable data carrier having stored thereon the program instructions or a data carrier signal having encoded thereon the program instructions.

In each case, the computer program product comprises instructions, which are loadable into the data-processing unit and adapted to cause execution of steps of any method described below or previously when run by the data-processing unit. According to the embodiments, the execution is entirely or partially achieved either on the controller 20, that is a single computer, or in a distributed system among several computers (notably via cloud computing).

More generally, the sleep analyzer 28 can advantageously be used in any context where the sleep quality is relevant due to their easy implementation. In addition, the person skilled in the art can consider any combination of the features of the previously mentioned embodiment of the sleep analyzer 28 to obtain new embodiment when the features are technically compatible. The scope of the invention remains defined by the appended claims.

## Claims

1. A system for monitoring (22) a sleep of a subject in a healthcare room, said system comprising a sleep analyzer (28) comprising:
- a housing (36),
- a sleep recorder (38) adapted to sense physical values relative to the sleep of said subject, the sleep recorder (38) including an electrode (62) adapted to record the electrical activity of the subject,
- a calculator (40) adapted to deduce a sleep state of the subject from the sensed physical values, said sleep state being **characterized by** distinct stages and states, including stage 1 of transition between being awake and being asleep, stage 2 of sleeping, stage 3 of deep slow wave sleep, stage R of rapid eye movement sleep, state of "pathological wakefulness" and state of "atypical sleep";
- a transceiver (42) adapted to transmit a data obtained from the deduced state of the subject to an external receiver (32),
the sleep recorder (38), the calculator (40) and the transceiver (42) being in the housing (36),
wherein the system for monitoring further comprises:
- a receiver (32) adapted to receive the data obtained from the deduced state of the subject, and
- an interface unit (34) adapted to indicate at least one of the received data, wherein said interface unit (34) is adapted to provide a symbol or color corresponding to said "do not wake up" signal or to said "awake" signal, wherein the calculator (40) is further configured to:
(i) identify successive sleep episodes of the subject from the deduced sleep state and classify each episode into at least three duration categories including: very short episodes of less than 5 minutes, medium episodes of between 5 and 10 minutes, and long episodes of more than 10 minutes; and
(ii) generate the "do not wake up" when the sleep state is in stage 3 or when the sleep state is in stage 2 over more than 10 minutes, and to suppress the emission of an "awake" signal during intra-sleep arousal episodes classified as very short episodes of less than 5 minutes;
such that medical personnel refrain from entering the healthcare room when the "do not wake up" signal is indicated.

2. The system for monitoring according to claim 1, wherein the sleep analyzer (28) comprises a sensor (58) for sensing a parameter relative to an environment of the sleep analyzer (28).

3. The system for monitoring according to any of claim 1 to 2, wherein the sleep analyzer (28) comprises a sensor (56) for sensing a parameter relative to the subject, notably to a breath of the subject, a movement of the subject or a brain activity of the subject.

4. The system for monitoring according to any one of claims 1 to 3, wherein the sleep analyzer (28) is provided with a power supply (44), the power supply (44) being in the housing (36).

5. The system for monitoring according to any one of claims 1 to 4, wherein the interface unit (34) is a human machine interface.

6. The system for monitoring according to any one of claims 1 to 5, wherein the interface unit (34) comprises at least one of a tablet (84) and an indicator (86).

7. A healthcare room (12), notably an intensive healthcare room (12), equipped with a system for monitoring (22) the sleep of a subject according to any one of claims 1 to 6.

8. A healthcare installation (10), notably an intensive healthcare installation (10), comprising several healthcare rooms (12), at least one of the healthcare room (12) being according to claim 7.

9. A method for monitoring a sleep of a subject in a healthcare room with a system for monitoring (22) the sleep of the subject according to any of claims 1 to 6, the method for monitoring comprising:
- sensing physical values relative to the sleep of the subject by using the sleep recorder (38), the sensing comprising recording the electrical activity of the subject with the electrode (62),
- deducing the sleep state of the subject from the sensed physical values by the calculator (40), said sleep state being **characterized by** distinct stages and states, including stage 1 of transition between being awake and being asleep, stage 2 of sleeping, stage 3 of deep slow wave sleep, stage R of rapid eye movement sleep, state of "pathological wakefulness" and state of "atypical sleep",
- transmitting with the transceiver (42) a data obtained from the deduced state of the subject to an external receiver (32), said data corresponding to a "do not wake up" signal, at least when the sleep state is in stage 3 or when the sleep state is in stage 2 over more than 10 minutes, or to an "awake" signal,
- receiving the data obtained from the deduced state of the subject by the receiver (32), and
- providing a symbol or color corresponding to said "do not wake up signal" or to said "awake" signal with the interface unit (34).

## Patentansprüche

1. System zur Überwachung (22) eines Schlafs eines Subjekts in einem Pflegeraum, wobei das System einen Schlafanalysator (28) umfasst, der Folgendes umfasst:
- ein Gehäuse (36),
- ein Schlafaufzeichnungsgerät (38), das dazu angepasst ist, physikalische Werte in Bezug auf den Schlaf des Subjekts zu erfassen, wobei das Schlafaufzeichnungsgerät (38) eine Elektrode (62) umfasst, die dazu angepasst ist, die elektrische Aktivität des Subjekts aufzuzeichnen,
- einen Rechner (40), der dazu angepasst ist, einen Schlafzustand des Subjekts aus den erfassten physikalischen Werten abzuleiten, wobei der Schlafzustand durch unterschiedliche Stadien und Zustände gekennzeichnet ist, einschließlich Stadium 1 des Übergangs zwischen Wachsein und Einschlafen, Stadium 2 des Schlafens, Stadium 3 des tiefen langsamwelligen Schlafs, Stadium R des Schlafs mit schnellen Augenbewegungen, Zustand der "pathologischen Wachheit" und Zustand des "atypischen Schlafs";
- einen Sendeempfänger (42), der dazu angepasst ist, Daten, die aus dem abgeleiteten Zustand des Subjekts erhalten wurden, an einen externen Empfänger (32) zu übertragen,
wobei das Schlafaufzeichnungsgerät (38), der Rechner (40) und der Sendeempfänger (42) sich im Gehäuse (36) befinden,
wobei das System zur Überwachung ferner Folgendes umfasst:
- einen Empfänger (32), der dazu angepasst ist, die Daten, die aus dem abgeleiteten Zustand des Subjekts erhalten wurden, zu empfangen, und
- eine Schnittstelleneinheit (34), die dazu angepasst ist, mindestens eines der empfangenen Daten anzuzeigen, wobei die Schnittstelleneinheit (34) dazu angepasst ist, ein Symbol oder eine Farbe bereitzustellen, die dem "Nicht-wecken"-Signal oder dem "Wach"-Signal entspricht, wobei der Rechner (40) ferner so konfiguriert ist, dass er:
(i) aufeinanderfolgende Schlafepisoden des Subjekts aus dem abgeleiteten Schlafzustand identifiziert und jede Episode in mindestens drei Dauerkategorien einteilt, einschließlich: sehr kurze Episoden von weniger als 5 Minuten, mittlere Episoden von zwischen 5 und 10 Minuten und lange Episoden von mehr als 10 Minuten; und
(ii) das "Nicht-wecken" erzeugt, wenn sich der Schlafzustand in Stadium 3 befindet oder wenn sich der Schlafzustand in Stadium 2 über mehr als 10 Minuten befindet, und die Abgabe eines "Wach"-Signals während Weckreaktionsepisoden innerhalb des Schlafs, die als sehr kurze Episoden von weniger als 5 Minuten eingestuft sind, unterdrückt;
wodurch medizinisches Personal davon absieht, den Pflegeraum zu betreten, wenn das "Nicht-wecken"-Signal angezeigt wird.

2. Das System zur Überwachung nach Anspruch 1, wobei der Schlafanalysator (28) einen Sensor (58) zum Erfassen eines Parameters in Bezug auf eine Umgebung des Schlafanalysators (28) umfasst.

3. Das System zur Überwachung nach einem der Ansprüche 1 bis 2, wobei der Schlafanalysator (28) einen Sensor (56) zum Erfassen eines Parameters in Bezug auf das Subjekt, insbesondere in Bezug auf eine Atmung des Subjekts, eine Bewegung des Subjekts oder eine Gehirnaktivität des Subjekts, umfasst.

4. Das System zur Überwachung nach einem der Ansprüche 1 bis 3, wobei der Schlafanalysator (28) mit einer Stromversorgung (44) versehen ist, wobei die Stromversorgung (44) sich in dem Gehäuse (36) befindet.

5. Das System zur Überwachung nach einem der Ansprüche 1 bis 4, wobei die Schnittstelleneinheit (34) eine Mensch-Maschine-Schnittstelle ist.

6. Das System zur Überwachung nach einem der Ansprüche 1 bis 5, wobei die Schnittstelleneinheit (34) mindestens eines von einem Tablet (84) und einem Anzeigeelement (86) umfasst.

7. Pflegeraum (12), insbesondere Intensivpflegeraum (12), ausgestattet mit einem System zur Überwachung (22) eines Schlafs eines Subjekts nach einem der Ansprüche 1 bis 6.

8. Pflegeeinrichtung (10), insbesondere Intensivpflegeeinrichtung (10), umfassend mehrere Pflegeräume (12), wobei mindestens einer der Pflegeräume (12) nach Anspruch 7 ist.

9. Verfahren zur Überwachung eines Schlafs eines Subjekts in einem Pflegeraum mit einem System zur Überwachung (22) des Schlafs des Subjekts gemäß einem der Ansprüche 1 bis 6, wobei das Verfahren zur Überwachung Folgendes umfasst:
- Erfassen physikalischer Werte in Bezug auf den Schlaf des Subjekts unter Verwendung des Schlafaufzeichnungsgeräts (38), wobei das Erfassen das Aufzeichnen der elektrischen Aktivität des Subjekts mit der Elektrode (62) umfasst,
- Ableiten des Schlafzustands des Subjekts aus den erfassten physikalischen Werten durch den Rechner (40), wobei der Schlafzustand durch unterschiedliche Stadien und Zustände gekennzeichnet ist, einschließlich Stadium 1 des Übergangs zwischen Wachsein und Einschlafen, Stadium 2 des Schlafens, Stadium 3 des tiefen langsamwelligen Schlafs, Stadium R des Schlafs mit schnellen Augenbewegungen, Zustand der "pathologischen Wachheit" und Zustand des "atypischen Schlafs",
- Übermitteln mit dem Sendeempfänger (42) von Daten, die aus dem abgeleiteten Zustand des Subjekts erhalten wurden, an einen externen Empfänger (32), wobei die Daten einem "Nicht-wecken"-Signal entsprechen, zumindest wenn sich der Schlafzustand in Stadium 3 befindet oder wenn sich der Schlafzustand in Stadium 2 über mehr als 10 Minuten befindet, oder einem "Wach"-Signal,
- Empfangen der aus dem abgeleiteten Zustand des Subjekts erhaltenen Daten durch den Empfänger (32) und
- Bereitstellen eines Symbols oder einer Farbe, die dem "Nicht-wecken"-Signal oder dem "Wach"-Signal entspricht, mit der Schnittstelleneinheit (34).

## Revendications

1. Système de surveillance (22) du sommeil d'un sujet dans une chambre de soins, ledit système comprenant un analyseur de sommeil (28) comprenant :
- un boîtier (36),
- un enregistreur de sommeil (38) adapté pour détecter des valeurs physiques relatives au sommeil dudit sujet, l'enregistreur de sommeil (38) incluant une électrode (62) adaptée pour enregistrer l'activité électrique du sujet,
- un calculateur (40) adapté pour déduire un état de sommeil du sujet à partir des valeurs physiques détectées, ledit état de sommeil étant **caractérisé par** des stades et états distincts, incluant le stade 1 de transition entre l'état d'éveil et l'état d'endormissement, le stade 2 de sommeil, le stade 3 de sommeil profond à ondes lentes, le stade R de sommeil à mouvements oculaires rapides, l'état d'« éveil pathologique » et l'état de « sommeil atypique » ;
- un émetteur-récepteur (42) adapté pour transmettre une donnée obtenue à partir de l'état déduit du sujet à un récepteur externe (32),
l'enregistreur de sommeil (38), le calculateur (40) et l'émetteur-récepteur (42) étant dans le boîtier (36),
dans lequel le système de surveillance comprend en outre :
- un récepteur (32) adapté pour recevoir la donnée obtenue à partir de l'état déduit du sujet, et
- une unité d'interface (34) adaptée pour indiquer au moins l'une des données reçues, dans laquelle ladite unité d'interface (34) est adaptée pour fournir un symbole ou couleur correspondant audit signal « ne pas réveiller » ou audit signal « éveillé », dans laquelle le calculateur (40) est en outre configuré pour :
(i) identifier des épisodes de sommeil successifs du sujet à partir de l'état de sommeil déduit et classer chaque épisode dans au moins trois catégories de durée incluant : des épisodes très courts de moins de 5 minutes, des épisodes moyens compris entre 5 et 10 minutes, et des épisodes longs de plus de 10 minutes ; et
(ii) générer le « ne pas réveiller » lorsque l'état de sommeil est au stade 3 ou lorsque l'état de sommeil est au stade 2 pendant plus de 10 minutes, et supprimer l'émission d'un signal « éveillé » pendant des épisodes d'éveil intra-sommeil classés comme épisodes très courts de moins de 5 minutes ;
de telle sorte que le personnel médical s'abstienne de pénétrer dans la chambre de soins lorsque le signal « ne pas réveiller » est indiqué.

2. Le système de surveillance selon la revendication 1, dans lequel l'analyseur de sommeil (28) comprend un capteur (58) pour détecter un paramètre relatif à un environnement de l'analyseur de sommeil (28).

3. Le système de surveillance selon l'une quelconque des revendications 1 à 2, dans lequel l'analyseur de sommeil (28) comprend un capteur (56) pour détecter un paramètre relatif au sujet, notamment une respiration du sujet, un mouvement du sujet ou une activité cérébrale du sujet.

4. Le système de surveillance selon l'une quelconque des revendications 1 à 3, dans lequel l'analyseur de sommeil (28) est pourvu d'une alimentation électrique (44), l'alimentation électrique (44) étant dans le boîtier (36).

5. Le système de surveillance selon l'une quelconque des revendications 1 à 4, dans lequel l'unité d'interface (34) est une interface homme-machine.

6. Le système de surveillance selon l'une quelconque des revendications 1 à 5, dans lequel l'unité d'interface (34) comprend au moins l'un d'une tablette (84) et d'un indicateur (86).

7. Chambre de soins (12), notamment chambre de soins intensifs (12), équipée d'un système de surveillance (22) d'un sommeil d'un sujet selon l'une quelconque des revendications 1 à 6.

8. Installation de soins (10), notamment installation de soins intensifs (10), comprenant plusieurs chambres de soins (12), au moins l'une de la chambre de soins (12) étant selon la revendication 7.

9. Procédé de surveillance du sommeil d'un sujet dans une chambre de soins avec un système de surveillance (22) du sommeil du sujet selon l'une quelconque des revendications 1 à 6, le procédé de surveillance comprenant :
- la détection de valeurs physiques relatives au sommeil du sujet en utilisant l'enregistreur de sommeil (38), la détection comprenant l'enregistrement de l'activité électrique du sujet avec l'électrode (62),
- la déduction de l'état de sommeil du sujet à partir des valeurs physiques détectées par le calculateur (40), ledit état de sommeil étant **caractérisé par** des stades et états distincts, incluant le stade 1 de transition entre l'état d'éveil et l'état d'endormissement, le stade 2 de sommeil, le stade 3 de sommeil profond à ondes lentes, le stade R de sommeil à mouvements oculaires rapides, l'état d'« éveil pathologique » et l'état de « sommeil atypique »,
- la transmission avec l'émetteur-récepteur (42) d'une donnée obtenue à partir de l'état déduit du sujet à un récepteur externe (32), ladite donnée correspondant à un signal « ne pas réveiller », au moins lorsque l'état de sommeil est au stade 3 ou lorsque l'état de sommeil est au stade 2 pendant plus de 10 minutes, ou à un signal « éveillé »,
- la réception de la donnée obtenue à partir de l'état déduit du sujet par le récepteur (32), et
- la fourniture d'un symbole ou couleur correspondant audit signal « ne pas réveiller » ou audit signal « éveillé » avec l'unité d'interface (34).
